Europäisches Patentamt

European Patent Office

Office européen des brevets

(11) Publication number: **0 425 270 A2**

# EUROPEAN PATENT APPLICATION

(21) Application number: **90311655.6**

(22) Date of filing: **24.10.90**

(51) Int. Cl.5: **B32B 27/18**

(30) Priority: **25.10.89 US 426832**

(43) Date of publication of application:
**02.05.91 Bulletin 91/18**

(84) Designated Contracting States:
**BE DE FR GB IT NL**

(71) Applicant: **HOECHST CELANESE
CORPORATION
Route 202-206 North
Somerville, New Jersey(US)**

(72) Inventor: **Kim, Dai W.
44 Huron Drive
Chatham, New Jersey(US)**

(74) Representative: **De Minvielle-Devaux, Ian
Benedict Peter et al
CARPMAELS & RANSFORD 43, Bloomsbury
Square
London WC1A 2RA(GB)**

(54) Super absorbent polymer composite structure.

(57) A super absorbent composite structure is disclosed which is comprised of a first porous layer, a foam containing super absorbent material second layer, an essentially liquid impermeable third layer and a means for adhering the first layer to the second layer and the second layer to the third layer. The composite structure can be of great use as a liquid absorbing material and can be of great utility in fabrics and materials or athletic equipment such as shoes, gloves, wrist bands, etc. where water absorbency and impermeability of the outer layer of the composite structure are important.

EP 0 425 270 A2

# SUPER ABSORBENT POLYMER COMPOSITE STRUCTURE

(b) Cross Reference to Related Applications

NONE

(c) Background of Invention

1. Field of invention

This invention relates to absorbent polymer articles and a process for their production. More particularly, this invention relates to a super absorbent composite structure comprised of a first porous wicking layer, a super absorbent foam second layer and a generally liquid impermeable third layer.

2. Prior Art

Recently there has been enhanced interest in producing increasingly absorbent materials, particularly for use in products such as sanitary napkins, diapers, disposable dust cloths, etc. Many of the prior art materials used to form these products have been non-woven fabrics, papers, pulps, spongy urethane resins, natural sponges and the like. However, these materials exhibit relatively low absorbency, thus failing to satisfy the need for a low volume, high absorbent material. Further, many of these prior art materials are not adaptable for use as composite structures for the one-way absorbency of fluids.

Substitutes for these materials such as cross-linked polyethylene oxides, cross-linked polyvinyl alcohols and hydrolyzed products of starch polyacrylonitrile grafted polymers have recently appeared on the market. While these products do show increased absorbency, they also suffer from significant disadvantages in that their absorbency is not sufficiently high to justify the high cost and difficult production of such products. In addition, some of these materials create disposal problems because they are not biologically degradable.

To solve this problem new and improved, highly absorbent products have been produced. For example, Japanese Kokai No. 57-92,032 (1982) discloses a polyurethane foam containing a water absorbing resin. This Kokai discloses the mixing of a conventional polyurethane foam with an absorbent resin referred to as "Sanwet IM-300-MP". The resultant product has improved water retaining and water stopping properties. While this Kokai discloses a highly absorbent polymer of the type that can be used within the composite structure of the instant invention, it fails to disclose the composite' structure used in this invention or the use of very fine, highly absorbent polymer particulates within its structure.

U.S. Patent Nos., 4,560,372 and 4,676,784 disclose disposable absorbent products in a layered structure comprised of an absorbent material mixed with a fibrous layer of resilient fibers composed of synthetic fibers such as polyesters, polyethylene, polypropylene and the like. Neither of these patents disclose the absorbent polymer nor the super absorbent composite structure of the instant invention.

U.S. Patent No. 4,076,663 discloses a biodegradable water absorbent polymer. While the polymer of this patent does show increased water absorbency, its use is quite limited.

U.S. Patent Nos. 4,454,268 4,337,181 and 4,133,784 disclose various types of films partially comprised of water absorbent polymers. While these patents disclose starch-based, water absorbent polymers prepared from the combination of starch and ethylene acrylic acid copolymers, they fail to disclose the particular type of absorbent polymer disclosed herein or the mixture of an absorbent polymer in a composite structure to produce the instant invention.

U.S. Patent No. 3,669,103 discloses water swellable, water insoluble polymeric sorbents for the absorption of aqueous fluids wherein said polymeric sorbents are lightly cross-linked polymers. This patent also discloses the use of a water insoluble polyurethane foam as a support for the polymeric absorbent. However, it fails to disclose the use of an absorbent polymer in general, the absorbent polymer disclosed herein or the use of an absorbent polymer in a composite structure.

U.S. Patent Nos. 4,731,391, 4,725,628 and 4,725,629 disclose processes for the production of a super absorbent polyurethane foam. The polyurethane foam is based on an interpenetrating polymer network of a cross-linked polyurethane and a substantially linear addition polymer containing a plurality of chain segments made of functional groups containing repeating units. The functional group of the repeating unit is selected from carbomoyl and carboxy substitutes and the alkali metal and ammonia salts thereof. Although these patents disclose an absorbent polyurethane foam, they fail to disclose the absorbent polymer disclosed herein or the super absorbent composite structure of this invention.

U.S. No. 4,713,069 discloses a breathable cloth-like barrier which is substantially impervious to liquid water but permeable to water vapor. This patent discloses a barrier especially well suited as the outer cover of certain absorbent articles.

U.S. Patent No. 4,726,976 discloses a composite substrate which is especially useful as a cover sheet for disposable article said substrate comprised of an inner polyethylene thermoplastic film secured to and between the inner surfaces of a top layer and a bottom layer of a non-woven polypropylene fibrous web.

Accordingly, it is an object of this invention to prepare a super absorbent composite structure.

It is a further object of this invention to prepare a super absorbent composite structure comprised of a first porous layer, a foam containing super absorbent material second layer, and a substantial liquid impermeable third layer.

These and other objects as well as the scope, nature and utilization of the invention will be apparent to those skilled in the art from the following detailed description and appended Claims.

## (e) Summary of Invention

In accordance with the present invention there is provided a super absorbent composite structure comprising a) a first porous cover sheet, b) a foam containing a super absorbent material second layer, and c) a substantially liquid impermeable third layer and d) a means to bind the first and second layers and the second and third layer together to form a super absorbent composite structure.

The product produced by this process can be highly useful in those areas where high fluid absorbency of one side of the composite along with liquid impermeability on the other side is important such as for sanitary napkins, box linings, food packaging and general packaging material. In addition, these new composite structures may also be used in athletic footwear, athletic garments, gloves and other such material where leather or leather-like material is used. These products not only absorb moisture but keep the moisture away from the skin surface of an individual using the composite.

## (f) Detailed Description of Invention

### (1) First Porous Layer

The first layer of this composite structure is made of a flexible fabric which is permeable to liquid and can be bound easily to the foam material contained in the second layer of the composite. The fabric can be made from any of the well-known textile materials such as cotton, wool, rayon, acetate, acrylic, polypropylene, co-polypropylene, polyester, nylon etc. Preferred materials include polyesters, polypropylene and nylon. The fabric can be woven or knitted and with knitted fabrics, those particularly preferred are those made using the circular needle process. Knitted fabrics made from textured nylon are more particularly preferred because such fabrics have stretching, resilience and containment properties which are important for the manufacture of this first porous layer.

This first porous layer can also be made of a non-woven fabric such as those made by the chemical and mechanically bonding of dry-laid webs, by wet processing using modified paper making techniques or spinbonding techniques. Of the non-woven materials, spunbonded fabrics are preferred.

The material of the first porous layers may also be produced from combinations of porous materials such as combinations of polyester and cotton. Preferably the first fabric layer should exhibit good wicking qualities to transfer fluid through the first fabric layer to the second foam layer.

### (2) Second Foamed Layer

The second foam layer is a combination of a super absorbent polymer combined within a foam

3

component. Any type of polymer which can be foamed can be used as the support polymer for the super absorbent polymer. For example, cellular polystyrene, polyvinyl chloride, co-polymers of styrene N-acrylonitrile, and polyethylene foams can be manufactured by a conventional physical stabilization process. Cellular polystyrene, cellulose acetate, polyolefins and polyvinyl chloride foams can be manufactured by any conventional decompression expansion process. However, the most versatile foams are produced from methods utilizing a chemical stabilization process for the production of foams such as polyurethane, polyisocyanates, polyphenols, epoxy resins and silicon resins.

In a preferred embodiment non-rigid polyurethane foams are used in the production of the composite. While a vast array of foamed products may be produced from polyurethanes from soft thermoplastic elastomers to hard thermoset rigid foams, in a more preferred embodiment, flexible polyurethane foams are prepared.

The chemical ingredients of a polyurethane foam system are (1) a polyfunctional isocyanate and (2) a hydroxyl-containing polymer along with the catalysts necessary to control the rate and type of reaction and other additives to control the surface chemistry of the process. A number of competing reactions can occur when (1) and (2) are brought together but the rain product, is polyurethane.

Originally, carbon dioxide was generated in situ (by the reaction of isocyanate with water) as a blowing agent for both rigid and flexible polyurethane foams. It is still common practice to rely partly on this method of gas generation for flexible materials. In addition, rigid cellular polyurethanes are now produced using volatile liquids, usually halocarbons, as expanding agents, producing gas as the foaming mixture heats up. These materials remain in the foam, and their presence as gas in the cells lowers the thermal conductivity of the foam considerably.

The general method of producing a cellular polyurethane is to mix the above ingredients and adjust the conditions and reactants such that the exotherm of the reaction causes expansion of the blowing agent and resultant foaming of the resins. The change of physical properties of the mixture is timed to coincide with the expansion of the foaming mixture so that dimensional stabilization by cross-linking of the polymer occurs at the time corresponding to minimum density.

The physical properties of the final cellular material can be varied broadly by controlling the degree of cross-linking in the final polymer. The average molecular weight between cross-links is generally 400-700 for rigid polyurethane foams, 700-2,500 for semi-rigid foams, and 2,500-20,000 for flexible polyurethane foams. The variation of cross-link density is normally obtained by varying the structure of the hydroxyl-containing component, since the structure of the diisocyanate is generally limited to some 6 or 8 commercially available compounds.

Because the variation between cross links is controlled by the structure of the polyhydroxy resin component, it is common to use the equivalent weight per hydroxyl unit in the resin as a criterion for rigid, semi-rigid, and flexible foams. In these terms, the equivalent weights of resins used for rigid foams vary from 100 to 150, for semi-rigid between 200 and 1,000, and for flexible from 1,000 to 10,000. The equivalent weight is defined as the ratio of molecular weight to hydroxyl functionality of the polyhydroxy resin. Two general types of processes have been developed for producing cellular polyurethanes on a commercial scale. These two processes are commonly called the one-shot process and the prepolymer processes. In the one-shot process all the necessary ingredients for producing the foam are mixed and then discharged from the mixer onto a suitable surface. The reactions begin immediately and proceed at such a rate that expansion starts quickly. The entire expansion is completed in 1 to 2 minutes. Curing may take several days.

In the prepolymer process the polyhydroxy component reacts with enough polyisocyanate to form a prenolymer with isocyanate end groups plus excess isocyanate. The prepolymer mixture then reacts with water to release carbon dioxide for expansion and to link the chains into a cross-linked matrix. This method is sometimes used for flexible foams.

In a semiprepolymer process, which has become more extensively used, a prepolymer containing excess isocyanate is mixed with a polyhydroxy resin and a separate blowing agent such as a halocarbon. In this case the prepolymer may contain only a few percent of the total polyhydroxy resin. This method is particularly useful for producing rigid foams.

After mixing, the resin can be dispensed by several different method. Typical methods are (1) as an expandable liquid, (2) as a spray of small droplets of mixed resins which adhere to surfaces and foam on these surfaces, and (3) as a froth into which some gas has been mixed prior to exiting from the mixing head, which causes the liquid mixture to froth as its pressure is decreased to atmospheric pressure. A modification of method (3) allows the froth to be sprayed in chunks upon a surface with subsequent additional expansion during curing. These flexible foams are based on polypropylene diols of 2000 molecular weight and triols up to 4000. The common diisocyanate reactants are 2,4,-toluene diisocyanate, methylene, bis(4-phenyl isocyanate), and polymethylene polyvinyl isocyanate. The diol is usually a polyether polyol prepared by the ring opening polymerization of a cyclic ether in the presence of water, i.e., the polyether has OHN- groups and acts like a simple diol. Alternatively a polyester polyol is used.

## (3) Super Absorbent Polymer Addition.

Prior to foaming of the foamable polymer, super absorbent polymer particulates are added to the foamable polymer to enhance the fluid absorption capability of the resulting foam product. Additional products may also be added to the foamable polymer besides the super absorbent polymer, such as surfactants, fillers or non-woven fibers to further enhance its properties. For example, in a preferred embodiment to assist in surface absorption, surfactants such as Pluronic-type surfactants may be added prior to the foaming operation. These products will enhance the fluid absorbance capability of the foams. The super absorbent polymers are solid, water insoluble but water swellable polymers which are capable of absorbing many times their own weight of water or aqueous solutions. These products are polymers of water soluble acrylic or vinyl monomers which are slightly cross-linked with a polyfunctional reactant. Such cross-linked polymers include polyvinylpyrrolidone, sulfonated polystyrene, polysulfoethyl acrylate, poly(2-hydroxyethylacrylate), polyacrylamide, polyacrylic acid, partial and complete alkali metal salts of polyacrylic acid, and the like. Also included are starch modified polyacrylic acids and hydrolyzed polyacrylonitrile and their alkali metal salts.

Useful super absorbent polymers can be made by polymerizing acrylic acid and starch in an aqueous medium using a polyfunctional monomer, e.g., N,N-alkylene-bis-acrylamide, as the crosslinking agent. This process is described in U.S. Patent No. 4,076,663, which is incorporated by reference. Super absorbent polymers can also be made as described in U.S. Patent No. 4,340,706 by the inverse polymerization of acrylic acid followed by crosslinking with a polyfunctional component, e.g., epichlorohydrin. Other super absorbent polymers and processes for their manufacture are disclosed in U.S. Patents Nos. 4,654,039; 3,669,103 and 3,670,731. All of the aforesaid patents are hereby incorporated by reference.

The super absorbent polymers particularly useful in this invention are those described in U.S. Patent No. 4,076,663. These super absorbent polymers have a particle sizes of from 0.5 micron to about 450 microns and are capable of absorbing at least about 15 times their weight of aqueous fluid. In a preferred embodiment superior absorption capabilities exist where the super absorbent polymer particles are less than about 20 microns in size. Particles of this size show absorbance capability in excess of 65 times their weight. In a preferred embodiment the super absorbent material is a graph co-polymer of about 91 percent acrylic acid and about 9 percent oxidized starch cross-linked with 0.1 percent N,N-methylene-bis-acrylamide.

These super absorbent polymer particles swell when they absorb aqueous fluid. The particles maintain the approximate shapes and geometry they had before contact with the fluid but the dimensions thereof are greatly enlarged.

In preparing the articles of this invention, the super absorbent polymers may also be mixed with other particulate matter which is insoluble in water and organic liquids but which is capable of absorbing or adsorbing liquids. One example of other particulate matter is naturally occurring cellulose materials, such as saw dust, crushed corncobs, cotton linters, wood pulp, and the like. Preferred particulate matter of this type are crushed or ground corncobs which can absorb up to 5 times their weight of aqueous solutions or organic liquids.

Another type of particulate matter useful in this invention is silica gel which can absorb fluids. Other useful absorbants are molecular-sieve zeolites, activated alumina and calcium sulfate, also known by the trade name Drierite.

Ion-exchange resins can also be used as the other particulate matter in combination with the cross-linked hydrocolloids in this invention. Particularly useful ion-exchange resins are the strong acid, cation exchange resins.

Other particulates which can be mixed with super absorbent polymer for use in this invention are clay materials, such as kaolin, montmorillonite, illite, vermiculite, glauconite, attapulgite and the like. These lay minerals are mixtures of metal oxides, e.g., aluminum oxide, magnesium oxide, potassium oxide, and silicon oxide, and generally exist in the amorphous state. Flocculated clay minerals, such as those described in U.S. Patent No. 3,935,363, which is hereby incorporated by reference, are particularly useful in this invention.

Once the super absorbent polymer is prepared in its particulate form, it is blended with the foamable polymer. When mixing the water absorbent polymer with the foamable polymer, the percentage of the water absorbent polymer in relation to the foamable polymer present may vary depending upon the degree of absorption of fluids that is desired. The greater the percentage of super absorbent polymer within the foam, the greater the absorbant capabilities of the foam. However, when the percentage of the super absorbent polymer approaches 90 percent, the structure of the foamed polymer begins to fall apart. Thus, in a preferred embodiment the percentage of super absorbent polymer within the super absorbent polymer/foamable polymer mixture should be in the range of about 2 to about 60 percent, and most preferably from about 2.5 to about 40 percent.

Once the super absorbent polymer is blended with the foamable polymer, any conventional foaming operation can be utilized to produce the foamed layer end product, including combining water with the super absorbent polymer and the foamable polymer. When a polyurethane prepolymer is used as the foamable polymer and water is employed as the initiator for the foaming operation, the water reacts with the isocyanate groups to cause cross linking and, as a by product, produces carbon dioxide which causes the foaming. Foaming may also be accomplished by blending in a low boiling halocarbon, such as ethylchloromethane or similar volatile materials.

Other processes for foaming other materials will depend upon the type of foamed end product that is desired. For example, an expandable formulation process or decompression expansion process can be used for the production of polystyrene or certain polyolefins. A frothing or dispersion process for the production of foams of latex rubber or urea-formaldehyde may also be used. The appropriate process will depend on the type of foamable polymer used and the end product sought.

Using the chemical formulation process with a foamable polyurethane prepolymer, the foaming of the ingredients begins immediately and concludes within about 1 to about 60 minutes depending upon the thickness of the foamed materials. To hasten the curing time, the foamed material can be heated. However, it is critical that the foamed polymer not be heated to a temperature above about 250°C since the preferred super absorbent polymer of this process begins to break down at that temperature. Therefore, in a preferred embodiment the curing temperature of the foamed polymer with water absorbent polymer within its structure is from about 150°C to about 250°C for about 1 to about 15 minutes.

Following the foaming of the polyurethane polymer, the skeletal structure of the foam can be modified to provide greater openings in the cell structure. Cell structure is commonly expressed as the fraction of open cells. When a large portion of the cells are interconnected by gas spaces, the foam has a large fraction of open cells, an open-celled foam. Conversely, a large proportion of non-interconnecting cells results in a small fraction of open cells, a closed cell foam. By modifying the fraction of open cells of the polyurethane foam, the absorbancy of the polyurethane foam can be increased or decreased as desired. Further, if the cells of the polyurethane foam contain the super absorbant polymer, the absorbancy of the polyurethane foam will increase by on even greater margin.

For example, Japanese Patent Application Kokai, No. 57-92,032 (1982) discloses a polyurethane foam that contains a water absorbant polymer wherein the percentage of the closed cells is in the range of 1 to 60 percent, wherein the diameter of the cells is in the range of 200 to 400 microns and the size of the water absorbant resin is in the range of 200 to 400 microns.

In conventional foaming operations the percentage of open cells may be as few as about 15 percent. To obtain increased open celled structure, the polyurethane foam are subjected to a thermal process whereby the windows or membranes are removed from the individual cell bubbles which make up the foam structure. See U.S. Patent Nos. 4,670,477, 3,171,820 and 3,175,025. When this procedure is allowed to go to completion, the percentage of open cells can increase to as high as 99 percent or higher. The choice of the percentages of open cells will depend on the needs or structure of the foam and can be modified to range from approximately 20 percent up to 99 percent or even greater. In general the open cell structure should be in excess of 80 percent and most preferably, in excess of 95 percent. In general, foams with over 95 percent void space are known as "Reticulated" foams. See Whittington's Dictionary of Plastics.

### 4) Third impermeable Layer

The third layer of the composite structure is made of a generally liquid impermeable material which is flexible. This layer is comprised of a flexible material which is impermeable to liquid but will assist in the retention of the super absorbent material in the second foamed layer. The third layer can be made in any of the well known barrier materials such as polyethylene or polypropylene film. See, for example, liquid impermeable material disclosed in U.S. Patent No. 4,731,066.

Certain "breathable" barriers may also be used which have been disclosed, for example in U.S. Patent No. 4,713,068. In addition, certain microporous materials, such as Celgard, manufactured by Hoechst-Celanese which contains microporous structures which allow the material to be liquid-impermeable while still permitting the release of certain gases from the second layer, are also allowable. See for example, U.S. Patent No. 3,156,242, 3,426,754, and 4,347,844.

In addition, certain well-known materials for use in athletic footwear or clothing such as leather may also be used as the third layer depending upon the product to be produced. In a preferred embodiment the material is a polypropylene non-woven fabric.

The three composite layers can be bound together by any conventional bonding procedure. For example, any conventional adhesive web or adhesive material which will allow the materials to retain their flexibility and water permeability can be used to bind the layers together. In a preferred embodiment a polyamide adhesive web is used to bind the layers.

After these three layers with the adhesive web are combined they are bound together by conventional procedures such as by placing them in a conventional heating press, heating them to a temperature of from about 100°C to 200°C and compressing them under sufficient pressure to allow the materials to be compressed from about 5 to about 50 percent. The temperature of heating, the time of heating and the percentage of compression will vary depending on the materials chosen to comprise the layers and the type of adhesive used. However, in a preferred embodiment when a polyamide adhesive is used, the composite product is heated to a temperature of about 130°C to 170°C for a period of about 5 to about 25 seconds under a pressure wherein the materials are compressed from about 5 to about 40 percent.

After the heating and compression step, the composite material is allowed to cool to room temperature. The composite formed by this process can absorb up to 65 times and preferable up to 200 times the weight of the super absorbent particulate in water or liquid solutions and 15 times and preferably up to 100 times the weight of the super absorbent particulate in a 1.0 percent saline solution and, in addition, will not leak under an applied pressure of up to about 80 p.s.i.

The articles of this invention can be used to absorb water or perspiration when used in athletic equipment, such as athletic shoes or gloves and can be used in commercial applications such as package lining, packing and where the absorption of fluids is important.

The following examples describe the invention in more detail. Percentages are by weight unless otherwise indicated.

## EXAMPLE 1

A conventional polyurethane foam formulation of toluene diisocyanate and a polyetherpolyol was modified by adding prior to the foaming process a sufficient amount of a super absorbent material to form a combination material containing 24 percent super absorbent material. The super absorbent material is a graph copolymer of 91 percent acrylic acid and 9 percent oxidized starch cross-linked with 0.1 percent N,N' methylene-bis-acrylamide made by the process described in U.S. Patent No. 4,076,663. The particle size of the super absorbent material was generally less than about 20 microns. A one-quarter inch thick polyurethane foam of approximately four inches by four inches was prepared using the above process.

On one side of the polyurethane foam was placed a similarly sized sixty percent/forty percent polyester/cotton terry cloth material. On the other side of the foam was placed a polypropylene non-woven material of approximately the same size. Placed between the polyester/cotton terry cloth material and the foam and between the foam and the polypropylene non-woven fabric were sheets of a polyamide spun web adhesive. On the outside of both sides of this composite material was placed a sheet of mylar to prevent the composite from sticking in the heating phase of the composite production.

This composite material was then placed between two hot plates maintained at temperatures between 120°C and 200°C and compressed down by about 20 percent of the original layer unit thickness for 10 seconds at which time the press was released. The material was then allowed to cool to air temperature.

The physical characteristics and water absorbency of this material was tested and is disclosed in attached Table 1.

## EXAMPLE 2

A composite structure similar to that produced in Example 1 was prepared except there was substituted for the polyester/cotton terrycloth material an acetate satin fabric and there was substituted for the polypropylene material a conventional finished leather material of approximately 70 mils in thickness. In addition, the polyurethane foam was 1/16th of an inch in thickness rather than 1/4th of an inch. The physical characteristics and water absorbency of this composite material are shown in Table 1.


EXAMPLE 3


A composite structure similar to that produced in Example 1 was prepared except the polyurethane foam was 1/8th of an inch in thickness. The physical characteristics and water absorbency of this composite material is shown in Table 1.


EXAMPLE 4


A composite structure similar to that produced in Example 2 was prepared except the polyurethane foam was 1/8th of an inch in thickness and instead of an acetate satan fabric, there was substituted a polypropylene non-woven fabric. The physical characteristics and water absorbency of this material is shown in attached Table 1.

TABLE I

| PHYSICAL PROPERTIES OF WATER ABSORBANT MATERIAL/POLYURETHANE FOAMS | | | | | |
|---|---|---|---|---|---|
| EXAMPLE | THICKNESS | WEIGHT | DENSITY | % ABSORPTION | |
| | (mls) | (oz/yd$^2$) | (LB/FT$^3$) | WATER | SALINE |
| 1 | 295 | 15.3 | 4.3 | 1248 | 873 |
| 2 | 157 | 54.3 | 25.8 | 285 | 233 |
| 3 | 190 | 12.6 | 5.5 | 1051 | 751 |
| 4 | 173 | 41.9 | 20.2 | 412 | 299 |

It is apparent from these examples that composite structures with a high degree of water absorbency can be be prepared comprised of a porous first layer, a foam containing a super absorbent material second layer and an essentially liquid impermeable third layer. These materials will find great utility in those products where high water absorbency of composite materials is required, such as for diapers, sanitary napkins and the like. In addition, these composites can be used in athletic material where perspiration absorbance is important such as sweat bands, athletic shoes and other such athletic clothing.


**Claims**

1. A super absorbent composite structure comprising (a) a first porous layer (b) a foam containing a super absorbent particulate material second layer (c) a substantially liquid impermeable third layer (d) and means for binding the first and second layers and the second and third layers together.

2. The super absorbent composite structure of Claim 1 wherein the foam is a polyurethane foam.

3. The super absorbent composite structure of Claim 1 or 2 wherein the porous layer is a non-woven polyester.

4. The super absorbent composite structure of Claim 1 or 2 wherein the porous layer is a cotton knit polyester fabric.

5. The super absorbent composite structure of any of Claims 1-4 wherein the essentially liquid impermeable third layer comprises leather, or nylon, or polypropylene.

6. The super absorbent composite structure of any of Claims 1-5 wherein the means for binding the first and second layers and the second and third layers together is an adhesive.

7. The super absorbent composite structure of Claim 6 wherein the adhesive is a polyamide adhesive.

8. The super absorbent composite structure of any of claims 1-7 wherein the amount of super absorbent particulate material within the foam is from 2 to 60 percent.

9. The super absorbent composite structure of any of Claims 1-8 wherein a surfactant is mixed with the super absorbent particulate material within the foam.

10. The super absorbent composite structure of any of Claims 1-9 wherein the super absorbent particulate material contained within the foam is less than 20 microns in size.

11. The super absorbent composite structure of claim 1 such that it absorbs moisture at least about 65 times the weight of the particulate in water, and at least 15 times the weight of the particulate in a 1 percent saline solution.

12. The super absorbent composite structure of any of Claims 1-11 wherein a surfactant is added to the super absorbent polymer prior to the foaming procedure.

13. The super absorbent composite structure of any of Claims 1-12 wherein the super absorbent material is a graft copolymer of approximately 91 percent acrylic acid and approximately 9 percent oxidized starch cross-linked with approximately 0.1 percent N,N′-methylene bis-acrylamide.

14. A super absorbent composite structure comprising a cotton/knit polyester fabric bound to a polyurethane foam containing a super absorbent particulate material which is bound to leather.

15. A super absorbent composite structure comprising a non-woven polyester bound to a polyurethane foam containing a super absorbent particulate material which is bound to a polypropylene layer.

16. A super absorbent composite structure comprising a cotton/knit layer bound to a polyurethane foam containing a super absorbent particulate material which is bound to an essentially liquid impermeable polypropylene.